# EUROPEAN PATENT SPECIFICATION

(11) **EP 3 995 530 B1**
(45) Date of publication and mention of the grant of the patent: **28.05.2025**
(21) Application number: 20868306.0
(22) Date of filing: 25.09.2020
(51) Int. Cl.: C08J 3/12, C08J 3/24, A61K 9/16, A61Q 19/00, A61K 8/02, A61K 8/86

(54) **A METHOD OF PREPARING POLYMER MICROPARTICLES, POLYMER MICROPARTICLES, MEDICAL COMPOSITION, COSMETIC COMPOSITION, MEDICAL ARTICLES, AND COSMETIC ARTICLES USING THE SAME**
VERFAHREN ZUR HERSTELLUNG VON POLYMERMIKROPARTIKELN, POLYMERMIKROPARTIKELN, MEDIZINISCHE ZUSAMMENSETZUNG, KOSMETISCHE ZUSAMMENSETZUNG, MEDIZINISCHE ARTIKELN UND KOSMETISCHE ARTIKELN DAMIT
PROCÉDÉ DE PRÉPARATION DE MICROPARTICULES POLYMÈRES, MICROPARTICULES POLYMÈRES, COMPOSITION MÉDICALE, COMPOSITION COSMÉTIQUE, ARTICLES MÉDICAUX ET ARTICLES COSMÉTIQUES LES COMPRENANT

(30) Priority: 27.09.2019 KR 20190120094; 24.09.2020 KR 20200124264
(43) Date of publication of application: 11.05.2022
(73) Proprietor: LG Chem, Ltd., Seoul 07336 (KR)
(72) Inventor: KIM, Yunseop, Daejeon 34122 (KR); SHIN, Jung Youn, Daejeon 34122 (KR); KIM, Jee Seon, Daejeon 34122 (KR); KIM, Chanjoong, Daejeon 34122 (KR); KIM, Minchae, Daejeon 34122 (KR); RYU, Je Young, Daejeon 34122 (KR)
(74) Representative: Goddar, Heinz J.
(86) International application number: PCT/KR2020/013129
(87) International publication number: WO 2021/060934

(56) References cited:
- WO-A1-2019/005848
- KR-A- 20070 004 159
- KR-A- 20160 002 256
- KR-A- 20160 025 026
- KR-A- 20160 027 095
- KR-A- 20170 090 965
- US-A1- 2004 127 698
- US-A1- 2018 215 840

## Description

### [TECHNICAL FIELD]

The present disclosure relates to a method of preparing polymeric microparticles that can realize excellent mechanical strength and stability as well as high crosslinking efficiency and production yield, polymeric microparticles, medical compositions, cosmetic compositions, medical articles and cosmetic articles comprising the same.

### [BACKGROUND OF THE INVENTION]

Along with the expansion of the fields of biopharmaceuticals and regenerative medicine, the need for the large-scale culture techniques capable of efficiently producing cells, tissues, microorganisms, and the like is growing.

Adherent cells are cultured using microcarriers within a 3D bioreactor. A cell, a culture medium, and a microcarrier are put in a bioreactor, and the cells are brought into contact with the microcarrier while stirring the culture medium, so that the cells are attached to the surface of the microcarrier and cultured. Since the microcarrier used at this time provides a high surface area/volume to which cells can attach and proliferate, it is suitable for large-scale culture of cells. However, when the adherent cells are expanded and cultured using a microcarrier, a process of recovering cells through the process of cell detachment is essentially involved after completion of cultivation. The cell detachment process induces cell detachment by using a proteolytic enzyme or changing the temperature. When such a detachment process is added, there is a problem that the manufacturing cost is increased, the economic efficiency is lowered, and cell damage may be induced.

The development of new materials and processes to solve these problems is steadily progressing. In particular, in the case of a cell therapeutic agent for injecting in vivo cells, attempts were made to ensure the biocompatibility of the microcarrier for culturing the cells and to omit the isolation and purification process. In this case, particles that implement the strength capable of withstanding the stress applied by fluid around the carrier during the culture process and after injection into the living body are necessary.

In addition, in the case of transdermal drug delivery technology in which a microcarrier that collects drugs or bioactive substances is mounted on a microneedle and delivered, the microcarrier must use a polymer suitable for applying to the living body, and it must have sufficient strength so that particles do not deform in the process of passing through the stratum corneum layer of the skin. Microcarriers that stably permeate the skin can deliver the loaded drug locally or systemically, allowing them to act on the required lesions.

Hyaluronic acid mainly used as a biocompatible substance is composed of N-acetyl-D-glucosamine and D-glucuronic acid, and is a biopolymer in which the repeating units are linearly linked. Hyaluronic acid is abundantly present in the vitreous humor of the eyeball, the synovial fluid of the joints, and cockscomb, and the like. Hyaluronic acid is commonly used as bioinjectable materials due to its excellent biocompatibility and viscoelasticity, but is in itself easily decomposed in vivo or under conditions such as acid or alkali, and thus, its use is limited. Further, when applied to microcarriers, there was a problem that it exhibits a negative surface charge in the pH range of a living body, and significantly reduces cell adhesion.

On the other hand, gelatin is a polymer obtained by hydrolyzing collagen, which is a bio-connective tissue, and is often used as a scaffold for cell culture. Gelatin can collect or culture the cells using its cell adhesion performance, but it is weak in the strength and has a phase transition characteristic according to temperature, so that researches were conducted to improve its strength by introducing a functional group by a chemical method.

Therefore, it is necessary to develop microcarriers or polymeric microparticles that are compatible with the living body and have excellent physical properties, such as physical strength, and stability against heat and enzymes.
US 2004/127698 A1 discloses a method for producing double-crosslinked hyaluronic material, comprising the steps of: (a) subjecting hyaluronic acid or a salt thereof to a first crosslinking reaction using either an epoxide compound or a carbodiimide compound as a crosslinking agent, and (b) subjecting the product obtained from step (a) to a second crosslinking reaction using the epoxide compound or carbodiimide compound not used in step (a) as a crosslinking agent, thereby obtaining a double crosslinking hyaluronate material.
WO2019/005848 A1 discloses a biocompatible tissue filler, comprising hyaluronic acid and an anaestetic agent, wherein a portion of the hyaluronic acid is modified by one or more linker moieties comprising one or more of an alkane or alkyl chain, an ether group, and a secondary alcohol, wherein the linker moieties are attached to the hyaluronic acid at one end of the linker.
US 2018/215840 A1 discloses a method of preparing a crosslinked hyaluronic acid having an elasticity of 50 to 200 Pa and a viscosity of 20 to 100 Pa, the method comprising crosslinking hyaluronic acid with an epoxy-based crosslinking agent having at least two epoxy functional groups in an ethanol-containing aqueous alkaline solution.

### [BRIEF SUMMARY OF THE INVENTION]

It is one object of the present disclosure to provide a method of preparing polymeric microparticles that can realize excellent mechanical strength and stability as well as high crosslinking efficiency and production yield.

It is another object of the present disclosure to provide polymeric microparticles prepared by the above-mentioned preparation method.

It is another object of the present disclosure to provide a medical composition comprising the above-mentioned polymeric microparticles.

It is yet another object of the present disclosure to provide a cosmetic composition comprising the above-mentioned polymeric microparticles.

It is yet another object of the present disclosure to provide a medical article comprising the above-mentioned medical composition.

It is a further object of the present disclosure to provide a cosmetic article comprising the above-mentioned cosmetic composition.

In order to achieve the above object, according to one aspect, there is provided a method of preparing polymeric microparticles comprising the steps of: subjecting a mixture containing a biocompatible polymer in an emulsion state and a first crosslinking agent to a primary crosslinking reaction to form crosslinked particles; and extracting the crosslinked particles and performing a secondary crosslinking reaction in an organic solvent phase including a second crosslinking agent,
wherein the step of subjecting a mixture containing a biocompatible polymer in an emulsion state and a first crosslinking agent to a primary crosslinking reaction to form crosslinked particles comprises adding an aqueous solution in which the biocompatible polymer is dissolved to a hydrophobic solvent to form an emulsion; and adding the first crosslinking agent to the mixed solution containing the emulsion,
wherein in the step of extracting the crosslinked particles and performing a secondary crosslinking reaction, the crosslinked particles and the second crosslinking agent proceed the secondary crosslinking reaction in a solid phase, and
wherein the biocompatible polymer means a polymer that can be injected directly into a human body for effective drug delivery of drugs applied to the human body.

According to another aspect, there is provided polymeric microparticles obtainable using the method of the invention comprising a polymer matrix in which a biocompatible polymer is crosslinked through a crosslinking agent,
wherein the polymeric matrix comprises a first crosslinking region in which the biocompatible polymer is crosslinked through a first crosslinking agent; and
a second crosslinking region in which the biocompatible polymer is crosslinked through a second crosslinking agent.

According to another aspect, there is provided a medical composition comprising the polymeric microparticles and a pharmaceutically effective substance contained in the polymeric microparticles.

According to yet another aspect, there is provided a cosmetic composition comprising the polymeric microparticles and a cosmetically effective substance contained in the polymeric microparticles.

According to yet another aspect, there is provided a medical article comprising the medical composition.

According to a further aspect, there is provided a cosmetic article comprising the cosmetic composition.

Further embodiments are disclosed in the dependent claims.

### [DETAILED DESCRIPTION OF THE INVENTION]

Hereinbelow, a method of preparing polymeric microparticles, polymeric microparticles, medical compositions, cosmetic compositions, medical articles and cosmetic articles comprising the same according to specific embodiments of the present disclosure will be described in more detail.

Unless otherwise specified throughout this specification, the technical terms used herein are only for reference to specific embodiments and is not intended to limit the present disclosure.

The singular forms "a", "an", and "the" used herein include plural references unless the context clearly dictates otherwise.

The term "including" or "comprising" used herein specifies a specific feature, region, integer, step, action, element and/or component, but does not exclude the presence or addition of a different specific feature, region, integer, step, action, element, component and/or group.

The terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

In the present disclosure, the (co)polymer means including both a polymer and a copolymer, the polymer means a homopolymer consisting of a single repeating unit, and the copolymer means a composite polymer containing two or more repeating units.

The molecular weight of the polymer used herein follows the measurement method of the manufacturer, and the method does not deviate from a conventional GPC measurement method. For example, the weight average molecular weight as used herein may mean a molecular weight measured by a light scattering method or a viscosity method.

As used herein, the microparticle means that the cross section of the particle is circular or elliptical, and the ratio (sphericity ratio) of minor axis/major axis of the particle is in the range of 0.7 to 1.0. The length of the minor axis and the major axis of the particles can be derived by taking an optical picture of the particles and calculating the average value of 30~100 arbitrary particles in an optical picture.

In the present disclosure, the particle diameter Dn means the diameter at n volume% point of cumulative distribution of the particle numbers depending on the particle diameter. That is, D50 is the particle diameter at 50% point of cumulative distribution of the particle numbers when the particle diameter of the particles are accumulated in ascending order, D90 is the particle diameter at 90% point of cumulative distribution of particle numbers depending on the particle diameter, and D10 is the particle diameter at 10% point of cumulative distribution of the particle numbers depending on the particle diameter.

The Dn can be measured by using a laser diffraction method. Specifically, the powder to be measured is dispersed in a dispersion medium and then introduced into a commercially available laser diffraction particle size measurement instrument (Horiba LA-960), a diffraction pattern difference according to the particle size is measured when the particles pass through a laser beam, and the particle size distribution is calculated. The particle diameter at points of 10%, 50% and 90% of the particle number cumulative distribution depending on the particle diameter can be calculated in a measuring device to thereby measure D10, D50, and D90. More specifically, in the present disclosure, the particle diameter may mean D50.

As used herein, the emulsion means a mixed phase in which one or more liquids that are immiscible with an oil phase or an aqueous phase are dispersed in another liquid (dispersion medium) in a particulate form (dispersoid). The emulsion can usually be divided into macroemulsions, microemulsions and nanoemulsions, depending on the particle size of the dispersed phase.

Now, the present disclosure will be described in more detail.

### 1. Method of preparing polymeric microparticles

According to one embodiment of the present disclosure, there can be provided a method of preparing polymeric microparticles comprising the steps of: subjecting a mixture containing a biocompatible polymer in an emulsion state and a first crosslinking agent to a primary crosslinking reaction to form crosslinked particles; and extracting the crosslinked particles and performing a secondary crosslinking reaction in an organic solvent phase including a second crosslinking agent,
wherein the step of subjecting a mixture containing a biocompatible polymer in an emulsion state and a first crosslinking agent to a primary crosslinking reaction to form crosslinked particles comprises adding an aqueous solution in which the biocompatible polymer is dissolved to a hydrophobic solvent to form an emulsion; and adding the first crosslinking agent to the mixed solution containing the emulsion,
wherein in the step of extracting the crosslinked particles and performing a secondary crosslinking reaction, the crosslinked particles and the second crosslinking agent proceed the secondary crosslinking reaction in a solid phase, and
wherein the biocompatible polymer means a polymer that can be injected directly into a human body for effective drug delivery of drugs applied to the human body.

The emulsion may mean a water-in-oil emulsion, an oil-in-water emulsion, an oil-in-oil emulsion, or a multiple emulsion. Specifically, the emulsion of the one embodiment may mean a water-in-oil type emulsion.

In conventional polymer fine particles, as the crosslinking reaction proceeds by adding the particles directly to an organic solvent, the morphology of the particles is greatly affected by the concentration of the polymer and the content of the crosslinking agent. In particular, when the concentration of the polymer is low or the content of the crosslinking agent is small, there was a problem that the morphology of the particles cannot be maintained and so it is difficult to form microparticles.

In view of the above, the present inventors have found through experiments that as the crosslinking reaction proceeds over two steps as in the method of preparing polymeric microparticles of the one embodiment, the crosslinking density is maximized even with the same amount of crosslinking agent and, at the same time, the mechanical strength and stability of microparticles are significantly improved, thereby completing the present disclosure.

In particular, it has also been found through experiments that as a secondary crosslinking reaction among the two-step crosslinking reaction proceeds in a solid phase, spherical particles can be realized even with a small amount of crosslinking agent, and the production yield is improved.

Specifically, the biocompatible polymer means a polymer that can be injected directly into a human body for effective drug delivery of drugs applied to the human body. Specifically, the biocompatible polymer may be at least one polymer selected from the group consisting of hyaluronic acid (HA), carboxymethyl cellulose (CMC), alginic acid, pectin, carrageenan, chondroitin (sulphate), dextran (sulfate), chitosan, polylysine, collagen, gelatin, carboxymethyl chitin, fibrin, agarose, pullulan, polylactide, polyglycolide (PGA), polylactide-glycolide copolymer (PLGA), polyanhydride, polyorthoester, polyetherester, polycaprolactone, polyesteramide, poly(butyric acid), poly(valeric acid), polyurethane, polyacrylate, ethylene-vinyl acetate polymer, acrylic substituted cellulose acetate, non-degradable polyurethane, polystyrene, polyvinyl chloride, polyvinyl fluoride, poly(vinyl imidazole), chlorosulphonate polyolefins, polyethylene oxide, polyvinylpyrrolidone (PVP), polyethylene glycol (PEG), polymethacrylate, hydroxypropylmethylcellulose (HPMC), ethyl cellulose (EC), hydroxypropyl cellulose (HPC), cyclodextrin, poly(N-isopropylamide) (PNIPAam), poloxamer, and polyacrylic acid copolymers, and derivatives thereof.

More specifically, the biocompatible polymer may be hyaluronic acid (HA), gelatin or a mixture of hyaluronic acid and gelatin.

As used herein, the hyaluronic acid may mean including both hyaluronic acid itself and a hyaluronic acid salt. Therefore, the hyaluronic acid aqueous solution may be a concept that includes both an aqueous solution of hyaluronic acid, an aqueous solution of a hyaluronic acid salt, and a mixed aqueous solution of hyaluronic acid and a hyaluronic acid salt. The salt of hyaluronic acid may be inorganic salts such as sodium hyaluronate, potassium hyaluronate, calcium hyaluronate, magnesium hyaluronate, zinc hyaluronate, cobalt hyaluronate, organic salts such as hyaluronic acid tetrabutylammonium, and mixtures thereof.

In one embodiment of the present disclosure, the molecular weight of hyaluronic acid is not particularly limited, but is preferably 500 g/mol or more and 5,000,000 g/mol or less in order to realize various physical properties and biocompatibility.

As used herein, the gelatin may mean a protein obtained by treating collagen derived from an animal with acid or alkali and subsequently extracting it.

In one embodiment of the present disclosure, the molecular weight of gelatin is not particularly limited, but is preferably 100,000 g/mol or more and 5,000,000 g/mol or less in order to realize various physical properties and biocompatibility.

In one embodiment of the present disclosure, examples of the first crosslinking agent are not particularly limited. Specifically, the first crosslinking agent may include one selected from the group consisting of 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone and glutaraldehyde.

In the present disclosure, the step of subjecting a mixture containing a biocompatible polymer in an emulsion state and a first crosslinking agent to a primary crosslinking reaction to form crosslinked particles includes adding an aqueous solution in which the biocompatible polymer is dissolved to a hydrophobic solvent to form an emulsion; and adding a first crosslinking agent to the mixed solution containing the emulsion.

That is, the primary crosslinking reaction may be a liquid phase reaction that proceeds by adding a first crosslinking agent to an emulsion containing a biocompatible polymer.

Further, in the step of subjecting a mixture containing a biocompatible polymer in an emulsion state and a first crosslinking agent to a primary crosslinking reaction to form crosslinked particles, the first crosslinking agent may be contained in an amount of 30 parts by weight or more and 300 parts by weight or less, 40 parts by weight or more and 250 parts by weight or less, or 50 parts by weight or more and 200 parts by weight or less with respect to 100 parts by weight of the biocompatible polymer. In the method of preparing polymeric microparticles according to one embodiment of the present disclosure, as a secondary crosslinking reaction proceeds after a primary crosslinking reaction as described above, polymeric microparticles that realize sufficient mechanical strength and sphericity ratio can be prepared even if a first crosslinking agent is added in a small amount of 30 parts by weight or more and 300 parts by weight or less with respect to 100 parts by weight of the biocompatible polymer.

When the first crosslinking agent is added in a content of less than 30 parts by weight with respect to 100 parts by weight of the biocompatible polymer, the degree of crosslinking is low, making it difficult to form particles, and when added in excess of 300 parts by weight, a technical problem may occur in which an excess of unreacted crosslinking agent is not purified but remains in the crosslinked particles.

On the other hand, according to the present disclosure, in the step of extracting the crosslinked particles and performing a secondary crosslinking reaction in an organic solvent phase including a second crosslinking agent, the crosslinked particles and the second crosslinking agent proceed a crosslinking reaction in a solid phase.

That is, according to the method of preparing the polymeric microparticles of the present disclosure, a secondary crosslinking reaction that proceeds in a solid phase in an alkaline organic solvent may be included. Further, the secondary crosslinking reaction proceeding in the solid phase may mean a secondary crosslinking reaction that dehydrates the swollen polymer particles and proceeds in a more aggregated state. As in one embodiment of the present disclosure, when the secondary crosslinking reaction proceeding in a solid phase in an organic solvent is included, that is, the secondary crosslinking reaction by dehydrating the swollen polymer particles and proceeding in a more aggregated state is included, excellent crosslinking efficiency is realized as compared with the case where it is not so, thereby capable of obtaining microparticles having high strength.

In one embodiment of the present disclosure, recovering the crosslinked particles may follow a method of using a mesh sieve having a sieve size of 30 µm or more. As described above, by recovering the crosslinked particles formed through the first crosslinking reaction, then redispersing them in an organic solvent and adding the second crosslinking agent, a secondary crosslinking can proceed in a more agglomerated solid phase.

In one embodiment of the present disclosure, examples of the second crosslinking agent are not particularly limited. Specifically, the second crosslinking agent may include one selected from the group consisting of 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone and glutaraldehyde. More specifically, the second crosslinking agent may be 1,4-butandiol diglycidyl ether (BDDE).

In one embodiment of the present disclosure, the first crosslinking agent and the second crosslinking agent may each independently include one selected from the group consisting of 1,4-butandiol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, sorbitol polyglycidyl ether, divinylsulfone and glutaraldehyde. Preferably, both the first crosslinking agent and the second crosslinking agent may be 1,4-butandiol diglycidyl ether (BDDE).

In addition, the second crosslinking agent may be contained in an amount of 30 parts by weight or more and 300 parts by weight or less, 40 parts by weight or more and 250 parts by weight or less, or 50 parts by weight or more and 200 parts by weight or less with respect to 100 parts by weight of the biocompatible polymer. In the method of preparing polymeric microparticles according to one embodiment of the present disclosure, as a secondary crosslinking reaction proceeds after a primary crosslinking reaction as described above, the second crosslinking agent is added in an amount of 30 parts by weight or more and 300 parts by weight or less with respect to 100 parts by weight of the biocompatible polymer, thereby capable of preparing polymeric microparticles realizing sufficient mechanical strength and sphericity ratio.

When the second crosslinking agent is added in an amount of less than 30 parts by weight with respect to 100 parts by weight of the biocompatible polymer, the degree of crosslinking is low, making it difficult to form particles, and when added in excess of 300 parts by weight, a technical problem may occur in which an excess of unreacted crosslinking agent is not purified but remains in the crosslinked particles.

On the other hand, in the step of extracting the crosslinked particles and performing a secondary crosslinking reaction in an organic solvent phase including a second crosslinking agent, the organic solvent may be one selected from the group consisting of ethanol, N,N-dimethylformamide, N,N-dimethylacetamide, N-methyl-2-pyrrolidone, N-methylcaprolactam, 2-pyrrolidone, N-ethylpyrrolidone, N-vinylpyrrolidone, dimethyl sulfoxide, tetramethylurea, pyridine, dimethyl sulfone, hexamethyl sulfoxide, gamma-butyrolactone, 3-methoxy-N,N-dimethylpropanamide, 3-ethoxy-N,N-dimethylpropanamide, 3-butoxy-N,N-dimethylpropanamide, 1,3-dimethyl-imidazolidinone, ethyl amyl ketone, methyl nonyl ketone, methyl ethyl ketone, methyl isoamyl ketone, methyl isopropyl ketone, cyclohexanone, ethylene carbonate, propylene carbonate, diglyme, 4-hydroxy-4-methyl-2-pentanone, ethylene glycol monomethyl ether, ethylene glycol monomethyl ether acetate, ethylene glycol monoethyl ether, ethylene glycol monoethyl ether acetate, ethylene glycol monopropyl ether, ethylene glycol monopropyl ether acetate, ethylene glycol monoisopropyl ether, ethylene glycol monoisopropyl ether acetate, ethylene glycol monobutyl ether, and ethylene glycol monobutyl ether acetate.

Further, in the step of extracting the crosslinked particles and performing a secondary crosslinking reaction in an organic solvent phase including a second crosslinking agent, the organic solvent phase including the second crosslinking agent may be an alkaline mixed solvent. That is, the secondary crosslinking reaction of the present disclosure can be performed in an organic solvent phase in which an alkaline aqueous solution is mixed with ethanol, which is an organic solvent. Examples of the alkaline aqueous solution is not so limited, but may be, for example, a sodium hydroxide aqueous solution.

As the organic solvent including the secondary crosslinking agent is an alkaline mixed solvent, it is possible to create an environment favorable for the nucleophilic substitution reaction (SN reaction) to increase the efficiency of the crosslinking reaction.

### 2. Polymeric microparticles

According to one embodiment of the present disclosure, there can be provided polymeric microparticles obtainable using the method of the invention comprising a polymer matrix in which a biocompatible polymer is crosslinked through a crosslinking agent,
wherein the polymeric matrix comprises a first crosslinking region in which the biocompatible polymer is crosslinked through a first crosslinking agent; and
a second crosslinking region in which the biocompatible polymer is crosslinked through a second crosslinking agent.

The present inventors proceeded with research on polymeric microparticles, and confirmed through experiments that by passing through a secondary crosslinking reaction that proceeds in a solid phase dehydrated and aggregated after a primary crosslinking reaction with a swollen emulsion as described above, the crosslinking density is maximized even with the same amount of crosslinking agent and, at the same time, the mechanical strength and stability of microparticles are significantly improved, thereby completing the present disclosure.

In particular, it has also been found through experiments that as a secondary crosslinking reaction among the two-step crosslinking reaction proceeds in a solid phase, spherical particles can be realized even with a small amount of crosslinking agent, and the production yield is improved.

Specifically, in the polymeric microparticles, the polymeric matrix includes a first crosslinking region in which the biocompatible polymer is crosslinked through a first crosslinking agent; and a second crosslinking region in which the biocompatible polymer is crosslinked through a second crosslinking agent.

The first crosslinking region means a crosslinking region formed by the progress of the primary crosslinking reaction of the emulsion containing the biocompatible polymer and the first crosslinking agent, and the second crosslinking region may mean a crosslinking region formed by the progress of the secondary crosslinking reaction with the second crosslinking agent without the progress of the primary crosslinking reaction with the first crosslinking agent in the emulsion containing the biocompatible polymer, and a crosslinking region in which the first crosslinking region and the second crosslinking agent are formed by an additional secondary crosslinking reaction.

On the other hand, the polymeric microparticles may have the average diameter in distilled water of 1 µm or more and 650 µm or less, 100 µm or more and 600 µm or less, or 200 µm or more and 550 µm or less, or 300 µm or more and 500 µm or less. When the average diameter of the polymeric microparticles satisfies the above-mentioned range, cell adhesion and culture performance are excellent.

Further, the polymeric microparticles may have a sphericity ratio of 0.9 or more and 1.0 or less, 0.95 or more and 1.0 or less, 0.96 or more and 0.99 or less, or 0.97 or more and 0.99 or less.

The sphericity ratio can be obtained by taking an optical photograph of the polymeric microparticles and calculating the average value of 30 to 100 arbitrary particles in the optical photograph.

Further, the polymeric microparticle may have a swelling degree of 55 or less, 0.1 or more and 55 or less, 0.1 or more and 50 or less, 0.2 or more and 50 or less, 0.5 or more and 30 or less, 0.8 or more and 18 or less according to the following Equation 1. Swelling degree = {(Average diameter in distilled water)3 - (Average diameter after drying)3}/(Average diameter after drying)3.

When the swelling degree of the polymeric microparticles exceeds 55, a technical problem may occur in which the crosslinking density is low, the mechanical strength is deteriorated, and the stability is reduced.

Further, the polymeric microparticles may have an average compressive strength of 0.1 mN or more, 0.1 mN or more and 100 mN or less, 0.11 mN or more and 100 mN or less, 1 mN or more and 100 mN or less, 1 mN or more and 90 mN or less, 1 mN or more and 60 mN or less, when deformed to a level of 50% of the average diameter.

The average compressive strength may be a value in which a compressive strength when deformed to a level of 50% of the average diameter for n polymeric microparticles is divided by n.

For example, in the present disclosure, the average compressive strength may be a value in which a compressive strength when deformed to a level of 50% of the average diameter for 30 polymeric microparticles is divided by 30.

When the average compressive strength of the polymeric microparticles is less than 0.1 mN, a technical problem may occur in which the mechanical strength of the polymeric microparticles is deteriorated and the cell culture ability is significantly reduced.

### 3. Medical composition

According to another embodiment of the present disclosure, there can be provided a medical composition comprising the polymeric microparticles of the other embodiment and a pharmaceutically effective substance contained in the polymeric microparticles. The content concerning the polymeric microparticles may include all the content described above in the other embodiments.

The pharmaceutically active substance may exist in a state contained in the polymeric microparticles.

Examples of the pharmaceutically effective substance are not particularly limited, and an effective substance suitable for the application can be applied without limitation depending on the application of the polymeric microparticles of the one embodiment. That is, specific examples of the pharmaceutically effective substance are not limited, and examples thereof may include a drug selected from the group consisting of ampetaminil, arecolin, atrophine, bupranolol, buprenorphine, capsaicin, carisoprodol, chlorpromazine, ciclopirox olamine, cocaine, desipramine, dyclonine, epinephrine, ethosuximide, floxetine, hydromorphine, imipramine, lidocaine, methamphetamine, melproic acid, methylpenidate, morphine, oxibutynin, nadolol, nicotine, nitroglycerin, pindolol, prilocaine, procaine, propanolol, rivastigmine, scopolamine, selegiline, tulobuterol, valproic acid, Donepezil and the like, and a peptide or protein-based drug selected from the group consisting of EPO (Erythropoietin), human growth hormone (hGH), Exenatide, GLP-1 (glucagon-like peptide-1), insulin, CSF (granulocyte colony-stimulating factor), estrogen, progesterone, parathyroid hormone (PTH), and the like. All pharmacological substances in which pharmacological effects are proven are applicable without limitation.

The amount of the pharmaceutically effective substance added is also not particularly limited, and the content can be used without limitation depending on the application use and target. For example, the effective substance can be contained in an amount of 0.0001 parts by weight or more and 1000 parts by weight or less with respect to 100 parts by weight of the polymeric microparticles. The effective substance may be contained in a small amount or an excessive amount without limitation with respect to the polymeric microparticles.

### 4. Cosmetic composition

According to yet another embodiment of the present disclosure, there can be provided a cosmetic composition comprising the polymeric microparticles of the other embodiment and a cosmetically effective substance contained in the polymeric microparticles. The content concerning the polymeric microparticles may include all the content described above in the other embodiments.

The cosmetically active substance may exist in a state contained in the polymeric microparticles.

Examples of the cosmetically effective substance are not particularly limited, and an effective substance suitable for the application can be applied without limitation depending on the application of the polymeric microparticles of the one embodiment.

The amount of the pharmaceutically effective substance added is also not particularly limited, and the content can be used without limitation depending on the application use and target. For example, the effective substance can be included in an amount of 0.0001 parts by weight or more and 1000 parts by weight or less with respect to 100 parts by weight of the polymeric microparticles. The effective substance may be contained in a small amount or an excessive amount without limitation with respect to the polymeric microparticles.

### 5. Medical article

According to yet another embodiment of the present disclosure, there can be provided a medical article comprising the medical composition of the other embodiment. The content concerning the medical composition may include all the content described above in the other embodiments.

Examples of the medical article are not particularly limited, and in order to realize the characteristics of the present disclosure, it is suitable when used by inserting it into the body, or when strength must be maintained for a long period of time. For example, the medical article may include a body implant, an implantable drug delivery system, a transdermal patch, a wound treatment, and the like.

### 6. Cosmetic article

According to a further embodiment of the present disclosure, there can be provided a cosmetic article comprising the cosmetic composition of the other embodiment. The content concerning the cosmetic composition may include all the content described above in the other embodiments.

Examples of the cosmetic article are not particularly limited, and in order to realize the characteristics of the present disclosure, for example, beauty creams, lotions, hair gels, packs (face masks) and the like can be mentioned.

Although the structure of the cosmetic pack is not particularly limited, it may include, for example, a support, and a cosmetically effective substance delivery layer formed on the support and including the polymeric microparticles of another embodiment. Examples of the support may include woven fabric, nonwoven fabric, silicone, polyethylene terephthalate, polyethylene, polypropylene, polyurethane, metal mesh, polyester, and the like.

### [Advantageous Effects]

According to the present disclosure, a method of preparing polymeric microparticles that can realize excellent mechanical strength and stability as well as high crosslinking efficiency and production yield, and polymeric microparticles using the same can be provided.

Hereinafter, the present disclosure will be described in more detail by way of examples.

### Example 1: Preparation of polymeric microparticles

0.15 g of hyaluronic acid salt (weight average molecular weight: 500 kDa, maker: SK Bioland) and 1 g of gelatin (gel strength: 300 g Bloom, maker: Sigma-Aldrich, product name: G2500) were respectively dissolved in distilled water at 3 wt.% and 20 wt.% to prepare two solutions, and these two solutions were mixed in a 1:1 volume ratio. This mixed solution (11.15 g) was mixed with a liquid paraffin solution (40 g) to prepare a mixed solution containing a water-in-oil (W/O) microemulsion. Then, 1.1 g (1 ml) of 1,4-butandiol diglycidyl ether (BDDE) as a crosslinking agent was added to the mixed solution, subjected to a crosslinking reaction at room temperature for 5 days, and then washed in the order of acetone, dichloromethane, and distilled water to prepare crosslinked particles. At this time, the crosslinked particles were recovered using a mesh sieve having a sieve size of 45 µm.

1.1 g (1 ml) of 1,4-butandiol diglycidyl ether (BDDE) was added to a mixed solution of 31.56 g of ethanol and 9.88 g of 0.1N aqueous NaOH solution to prepare a solution, and the crosslinked particles were added to this solution and subjected to a crosslinking reaction at room temperature for 3 days to prepare polymeric crosslinked particles. The prepared particles were washed in the order of ethanol and distilled water, and then the prepared crosslinked particles were recovered using a sieve with a mesh size of 45 µm. The recovered crosslinked particles were filtered using a mesh sieve with a sieve size of 300 µm, and the remaining crosslinked particles were analyzed.

### Example 2: Preparation of polymeric microparticles

Polymeric microparticles were prepared in the same manner as in Example 1, except that only 1 g of gelatin was added without adding hyaluronic acid salt.

### Comparative Example 1: Preparation of polymeric microparticles

0.15 g of hyaluronic acid salt (weight average molecular weight: 500 kDa, maker: SK Bioland) and 1 g of gelatin (gel strength: 300 g Bloom, maker: Sigma-Aldrich, product name: G2500) were respectively dissolved in distilled water at 3 wt.% and 20 wt.% to prepare two solutions, and then these two solutions were mixed in a 1:1 volume ratio. This mixed solution (11.15 g) was mixed with a liquid paraffin solution (40 g) to prepare a mixed solution containing a water-in-oil (W/O) microemulsion. Then, 1.1 g (1 ml) of 1,4-butandiol diglycidyl ether (BDDE) as a crosslinking agent was added to 1.1 g (1 ml) of the mixed solution, and subjected to a crosslinking reaction at room temperature for 5 days.

Then, 1.1 g (1 ml) of 1,4-butandiol diglycidyl ether (BDDE) as a crosslinking agent was added to 1.1 g (1 ml) of the mixed solution, and subjected to a crosslinking reaction at room temperature for 3 days to prepare polymeric microparticles. The prepared particles were washed in the order of acetone, dichloromethane, and distilled water, and then the crosslinked particles were recovered using a sieve with a mesh size of 45 µm. The recovered crosslinked particles were filtered using a sieve with a mesh size of 300 µm, and the remaining crosslinked particles were analyzed.

### Comparative Example 2: Preparation of polymeric microparticles

0.15 g of hyaluronic acid salt (weight average molecular weight: 500 kDa, maker: SK Bioland) and 1 g of gelatin (gel strength: 300 g Bloom, maker: Sigma-Aldrich, product name: G2500) were respectively dissolved in distilled water at 3 wt.% and 20 wt.% to prepare two solutions, and then these two solutions were mixed in a 1:1 volume ratio. This mixed solution (11.15 g) was mixed with a liquid paraffin solution (40 g) to prepare a mixed solution containing a water-in-oil (W/O) microemulsion. Then, 1.1 g (1 ml) of 1,4-butandiol diglycidyl ether (BDDE) as a crosslinking agent was added to 51.15 g of the mixed solution, and subjected to a crosslinking reaction at room temperature for 5 days. The prepared particles were washed in the order of acetone, dichloromethane, and distilled water, and then the crosslinked particles were recovered using a sieve having a mesh size of 45 µm.

The crosslinked particles were added to a mixed solution of 31.56 g of ethanol and 9.88 g of 0.1N NaOH aqueous solution, subjected to a crosslinking reaction at room temperature for 3 days to prepare polymeric microparticles. The prepared particles were washed in the order of ethanol and distilled water, and then the crosslinked particles were recovered using a sieve with a mesh size of 45 µm. The recovered crosslinked particles were filtered using a mesh sieve with a sieve size of 300 µm, and the remaining crosslinked particles were analyzed.

### Comparative Example 3: Preparation of polymeric microparticles

0.15 g of hyaluronic acid salt (weight average molecular weight: 500 kDa, maker: SK Bioland) and 1 g of gelatin (gel strength: 300 g Bloom, maker: Sigma-Aldrich, product name: G2500) were respectively dissolved in distilled water at 3 wt.% and 20 wt.% to prepare two solutions, and then these two solutions were mixed in a 1:1 volume ratio. This mixed solution (11.15 g) was mixed with a liquid paraffin solution (40 g) to prepare a mixed solution containing a water-in-oil (W/O) microemulsion. Then, 1.1 g (1 ml) of 1,4-butandiol diglycidyl ether (BDDE) as a crosslinking agent was added to 51.15 g of the mixed solution, and subjected to a crosslinking reaction at room temperature for 5 days. The prepared particles were washed in the order of acetone, dichloromethane, and distilled water, and then the crosslinked particles were recovered using a sieve having a mesh size of 45 µm. The recovered crosslinked particles were filtered using a sieve with a mesh size of 300 µm, and the remaining crosslinked particles were analyzed.

### Experimental Example: Measurement of physical properties of polymeric microparticles

For the polymeric microparticles prepared in the above Examples and Comparative Examples, the swelling degree, sphericity degree, strength, cell culture suitability, and stability of the polymeric microparticles were evaluated by the following methods.

### 1. Average diameter and swelling degree

The average diameter of the polymeric microparticles of Examples and Comparative Examples in distilled water was measured and the swelling degree was calculated therefrom.

The swelling degree of the particles according to the present disclosure was calculated according to the following Equation 1: Swelling degree = {(Average diameter in distilled water)3 - (Average diameter after drying)3}/(Average diameter after drying)3.

At this time, the closer the swelling degree value to 0 means that swelling does not occur, and the closure the value to 0 means that the particles are swelled and become larger.

The average diameter in distilled water was measured using a laser particle size analyzer (Horiba, Partica LA-960), and the average diameter after drying was calculated from the diameters of 30 arbitrary particles in the SEM (Hitach, S-4800) photograph of the dried microparticles.

### 2. Sphericity degree

Optical (Olympus, BX53) photographs of the polymeric microparticles of Examples and Comparative Examples were taken, and the degree of sphericity degree was calculated therefrom.

The degree of sphericity degree according to the present disclosure was calculated as the average value of the ratio of the longest diameter to the shortest diameter (major axis/minor axis ratio) of 30 arbitrary particles in the optical photograph.

At this time, as the sphericity degree value is closer to 1, it refers to the closer to the spherical shape.

### 3. Strength

For the polymeric microparticles of Examples and Comparative Examples, the strength of the microparticles was measured using a texture analyzer equipment. 30 microparticles swollen with distilled water were placed as a single layer on the region under the flat cylindrical probe of the equipment equipped with a 5 N load cell. The initial trigger force was set to 1 mN, and the particles were compressed at a rate of 1 mm/s. The force when the average particle diameter was deformed to a level of 50% of the original diameter was defined as the compressive force.

The average compressive strength is a value calculated by dividing the compressive force by 30, which is the number of microparticles to be measured.

### 4. Cell culture suitability

A cell culture medium was filled in a 6-well plate, polymeric microparticles and cells were added and the cells were cultured by plate-rock method. At this time, the temperature of the culture medium was maintained at 37°C, and the cells were cultured for 3 days, and the number of cells cultured with the polymeric microparticles was confirmed.

At this time, the cell culture suitability was evaluated based on the following criteria.
Suitable: when the number of cultured cells relative to the number of injected cells is 150% or more
Unsuitable: when the number of cultured cells relative to the number of injected cells is less than 150%

### 5. Stability

The stability of the polymeric microparticles placed in phosphate-buffered saline solution during the sterilization process using a high-temperature and high-pressure sterilizer (Autoclave) and the particle stability after long-term culture were evaluated based on the following criteria:
Suitable: when the weight reduction rate of the dried polymeric microparticles before and after use of autoclave is 10% or less
Unsuitable: when the weight reduction rate of the dried polymeric microparticles before and after use of autoclave exceeds 10%

**[Table 1]**

| Category | Example 1 | Example 2 | Comparative Example 1 | Comparative Example 2 | Comparative Example 3 |
|---|---|---|---|---|---|
| Average diameter in distilled water (*µ*m) | 323±24 | 471±10 | 482±62 | 497±12 | 498±16 |
| Average diameter after drying (*µ*m) | 123±11 | 386±24 | 125±25 | 121±15 | 123±21 |
| Swelling degree | 17.19 | 0.82 | 56.51 | 132.43 | 127.79 |
| Sphericity degree | 0.98±0.06 | 0.91±0.17 | 0.99±0.01 | 0.98±0.09 | 0.98±0.03 |
| Average compressive strength (mN) | 1.97 | 1.17 | 0.90 | 0.30 | 0.23 |
| Cell culture suitability | Suitable | Suitable | Suitable | Suitable | Suitable |
| Stability | Suitable | Suitable | Unsuitable | Unsuitable | Unsuitable |

As shown in Table 1, it could be confirmed that in the polymeric microparticles of Examples, which are prepared by the preparation method of the present disclosure comprising the step of performing a primary crosslinking, then recovering the particles and performing a secondary crosslinking, not only the number of cultured cells is equal to or higher than 150% compared to the number of cells injected first which is thus suitable for cell culture, but also the weight reduction rate of the dried polymeric microparticles before and after autoclave treatment appears to be 10% or less, which is thus suitable for sterilization treatment and long-term culture.

In addition, it could be confirmed that in the polymeric microparticles of Examples, the sphericity degree appears to be 0.9 or more, indicating a high sphericity degree, and at the same time, the swelling degree appears to be 0.82 or more and 17.19 or less, indicating an excellent degree of crosslinking. Further, it could be confirmed that the average compressive strength appears to be 1.17 mN or more and thus, excellent mechanical properties are realized and the particles have a high crosslinking density.

That is, it was confirmed that the polymeric microparticles of Examples, which are prepared by the present preparation method comprising the step of performing a primary crosslinking, then recovering the particles and performing a secondary crosslinking, are suitable for cell culture, sterilization treatment, and long-term culture, and also can realize excellent crosslinking density and mechanical properties.

On the other hand, it could be confirmed that in the polymeric microparticles of Comparative Example 1, which passed immediately through the secondary crosslinking without recovering the particles after the primary crosslinking, not only the weight reduction rate of the dried polymeric microparticles before and after autoclave treatment appears to be more than 10%, making it unsuitable for sterilization and long-term culture, but also the swelling degree appears to be 56.51, indicating inferior degree of crosslinking as compared with Examples of the present disclosure. Further, it could be confirmed that the average compressive strength appears to be 0.9 mN, indicating inferior mechanical properties, and the particles have a low crosslinking density.

In addition, it could be confirmed that in the polymeric microparticles of Comparative Example 2, which recovered the particles after the primary crosslinking and passed through only the dehydration process without the addition of the secondary crosslinking agent, not only the weight reduction rate of the dried polymeric microparticles before and after autoclave treatment exceeds 10%, making it unsuitable for sterilization and long-term culture, but also the swelling degree appear to be 132.43, indicating inferior degree of crosslinking as compared with Examples. Further, it could confirm that the average compressive strength is 0.3 mN, indicating inferior mechanical properties, and the particles have a low crosslinking density.

Further, it could be confirmed that in the polymeric microparticles of Comparative Example 3, which passed through only the primary crosslinking and omitted the secondary crosslinking step, not only the weight reduction rate of the dried polymeric microparticles before and after autoclave treatment appears to be more than 10%, making it unsuitable for sterilization and long-term culture, but also the swelling degree appears to be 127.79, indicating an inferior degree of crosslinking compared to Examples of the present disclosure. Further, it could confirm that the average compressive strength appears to be 0.23 mN, indicating inferior mechanical properties, and the particles have a low crosslinking density.

## Claims

1. A method of preparing polymeric microparticles comprising the steps of:
subjecting a mixture containing a biocompatible polymer in an emulsion state and a first crosslinking agent to a primary crosslinking reaction to form crosslinked particles; and
extracting the crosslinked particles and performing a secondary crosslinking reaction in an organic solvent phase including a second crosslinking agent,
wherein the step of subjecting a mixture containing a biocompatible polymer in an emulsion state and a first crosslinking agent to a primary crosslinking reaction to form crosslinked particles comprises adding an aqueous solution in which the biocompatible polymer is dissolved to a hydrophobic solvent to form an emulsion; and adding the first crosslinking agent to the mixed solution containing the emulsion,
wherein in the step of extracting the crosslinked particles and performing a secondary crosslinking reaction, the crosslinked particles and the second crosslinking agent proceed the secondary crosslinking reaction in a solid phase, and
wherein the biocompatible polymer means a polymer that can be injected directly into a human body for effective drug delivery of drugs applied to the human body.

2. Polymeric microparticles obtainable using the method of claim 1 comprising a polymer matrix in which a biocompatible polymer is crosslinked through a crosslinking agent,
wherein the polymeric matrix comprises a first crosslinking region in which the biocompatible polymer is crosslinked through a first crosslinking agent; and
a second crosslinking region in which the biocompatible polymer is crosslinked through a second crosslinking agent.

3. A medical composition comprising the polymeric microparticles of claim 2 and a pharmaceutically effective substance contained in the polymeric microparticles.

4. A cosmetic composition comprising the polymeric microparticles of claim 2 and a cosmetically effective substance contained in the polymeric microparticles.

5. A medical article comprising the medical composition of claim 3.

6. A cosmetic article comprising the cosmetic composition of claim 5.

## Patentansprüche

1. Verfahren zur Herstellung von polymeren Mikropartikeln, umfassend die Schritte:
Unterziehen einer Mischung, die ein biokompatibles Polymer in einem Emulsionszustand und ein erstes Vernetzungsmittel enthält, einer primären Vernetzungsreaktion, um vernetzte Partikel zu bilden; und
Extrahieren der vernetzten Partikel und Durchführen einer sekundären Vernetzungsreaktion in einer organischen Lösungsmittelphase, die ein zweites Vernetzungsmittel enthält,
wobei der Schritt des Unterziehens einer Mischung, die ein biokompatibles Polymer in einem Emulsionszustand und ein erstes Vernetzungsmittel enthält, einer primären Vernetzungsreaktion, um vernetzte Partikel zu bilden, das Zugeben einer wässrigen Lösung, in der das biokompatible Polymer gelöst ist, zu einem hydrophoben Lösungsmittel, um eine Emulsion zu bilden; und das Zugeben des ersten Vernetzungsmittels zu der gemischten Lösung, die die Emulsion enthält, umfasst,
wobei in dem Schritt des Extrahierens der vernetzten Partikel und Durchführens einer sekundären Vernetzungsreaktion die vernetzten Partikel und das zweite Vernetzungsmittel die sekundäre Vernetzungsreaktion in einer festen Phase fortführen, und
wobei das biokompatible Polymer ein Polymer bedeutet, das direkt in einen menschlichen Körper injiziert werden kann, zur effektiven Arzneimittelabgabe von Arzneimitteln, die auf den menschlichen Körper angewendet werden.

2. Polymere Mikropartikel, erhältlich unter Verwendung des Verfahrens nach Anspruch 1, umfassend eine Polymermatrix, in der ein biokompatibles Polymer durch ein Vernetzungsmittel vernetzt ist,
wobei die Polymermatrix einen ersten Vernetzungsbereich umfasst, in dem das biokompatible Polymer durch ein erstes Vernetzungsmittel vernetzt ist; und
einen zweiten Vernetzungsbereich, in dem das biokompatible Polymer durch ein zweites Vernetzungsmittel vernetzt ist.

3. Medizinische Zusammensetzung, umfassend die polymeren Mikropartikel nach Anspruch 2 und eine pharmazeutisch wirksame Substanz, die in den polymeren Mikropartikeln enthalten ist.

4. Kosmetische Zusammensetzung, umfassend die polymeren Mikropartikel nach Anspruch 2 und eine kosmetisch wirksame Substanz, die in den polymeren Mikropartikeln enthalten ist.

5. Medizinischer Artikel, umfassend die medizinische Zusammensetzung nach Anspruch 3.

6. Kosmetischer Artikel, umfassend die kosmetische Zusammensetzung nach Anspruch 5.

## Revendications

1. Procédé de préparation de microparticules polymères comprenant les étapes de:
soumettre un mélange contenant un polymère biocompatible dans un état d'émulsion et un premier agent de réticulation à une réaction de réticulation primaire pour former des particules réticulées; et
extraire les particules réticulées et effectuer une réaction de réticulation secondaire dans une phase de solvant organique contenant un deuxième agent de réticulation,
dans lequel l'étape de soumettre un mélange contenant un polymère biocompatible dans un état d'émulsion et un premier agent de réticulation à une réaction de réticulation primaire pour former des particules réticulées comprend l'ajout d'une solution aqueuse dans laquelle le polymère biocompatible est dissous dans un solvant hydrophobe pour former une émulsion; et l'ajout du premier agent de réticulation à la solution mixte contenant l'émulsion,
dans lequel dans l'étape d'extraire les particules réticulées et d'effectuer une réaction de réticulation secondaire, les particules réticulées et le deuxième agent de réticulation effectuent la réaction de réticulation secondaire dans une phase solide, et
dans lequel le polymère biocompatible signifie un polymère qui peut être injecté directement dans un corps humain pour l'administration efficace de médicaments appliqués au corps humain.

2. Microparticules polymères pouvant être obtenues en utilisant le procédé de la revendication 1 comprenant une matrice polymère dans laquelle un polymère biocompatible est réticulé par un agent de réticulation,
dans lequel la matrice polymère comprend une première région de réticulation dans laquelle le polymère biocompatible est réticulé par un premier agent de réticulation; et
une deuxième région de réticulation dans laquelle le polymère biocompatible est réticulé par un deuxième agent de réticulation.

3. Composition médicale comprenant les microparticules polymères de la revendication 2 et une substance pharmaceutiquement efficace contenue dans les microparticules polymères.

4. Composition cosmétique comprenant les microparticules polymères de la revendication 2 et une substance cosmétiquement efficace contenue dans les microparticules polymères.

5. Article médical comprenant la composition médicale de la revendication 3.

6. Article cosmétique comprenant la composition cosmétique de la revendication 5.
